# EUROPEAN PATENT SPECIFICATION

(11) **EP 3 456 810 B1**
(45) Date of publication and mention of the grant of the patent: **15.04.2020**
(21) Application number: 18194379.6
(22) Date of filing: 14.09.2018
(51) Int. Cl.: C12M 1/107, C12M 1/00, C10L 3/10, F25J 3/06, F25J 3/02

(54) **PLANT AND PROCESS FOR BIOGAS UPGRADING**
ANLAGE UND VERFAHREN ZUR BIOGASVEREDELUNG
INSTALLATION ET PROCÉDÉ DE VALORISATION DE BIOGAZ

(30) Priority: 18.09.2017 IT 201700103870
(43) Date of publication of application: 20.03.2019
(73) Proprietor: Criotec Impianti S.p.A., 10034 Chivasso (Torino) (IT)
(72) Inventor: ROVETA, Guido, I-10037 Torrazza Piemonte (Torino) (IT); MUSSINATTO, Adriano, I-10155 Torino (IT)
(74) Representative: Gerbino, Angelo

(56) References cited:
- EP-A1- 2 277 614
- WO-A1-2011/110322
- US-A- 3 254 496
- US-A1- 2011 132 034
- THOMAS E R ET AL: "CONCEPTUAL STUDIES FOR CO2/NATURAL GAS SEPARATION USING THE CONTROLLED FREEZE ZONE (CFZ) PROCESS", GAS SEPARATION & PURIFICATION, GUILDFORD, SURREY, GB, vol. 2, 1 June 1988 (1988-06-01), pages 84-89, XP001223902, DOI: 10.1016/0950-4214(88)80017-3

## Description

The present invention refers to a plant and a process for upgrading biogas.

As is well known, such term refers to a mixture of gases produced by the anaerobic bacterial fermentation of organic residues of vegetable and/or animal origin.

The most interesting component of biogas is methane, which must be separated from other components, in particular, carbon dioxide, and liquefied in order to be properly exploited, typically for transport purposes. Thomas et al. described (Gas Separation & Purification, vol. 2, 1988, pages 84-89) an installation to separate methane from impurities in a gaseous stream, comprising a separation column with two zones. The installation comprises a cooling unit to separate carbon dioxide, which is recycled to the separation column.

One object of the present invention is therefore to provide a plant and an operating procedure thereof, which allow liquefied methane to be obtained from biogas in a more efficient and economical way than those of the prior art.

Such object is achieved by means of a plant and the operating process thereof having the features indicated in the independent claims 1 and 6 below, respectively.

Preferred features of the plant and the process of the invention are described in the dependent claims.

The present invention utilizes a cryogenic technology not only to liquefy methane, but also to separate it from the other biogas components, thus allowing the plant to be simplified and its operational efficiency to be increased.

It should also be noted that - although liquefied methane is the main product of the process of the invention - the latter also allows liquid carbon dioxide to be obtained simultaneously with a level of purity suitable for use in an industrial environment.

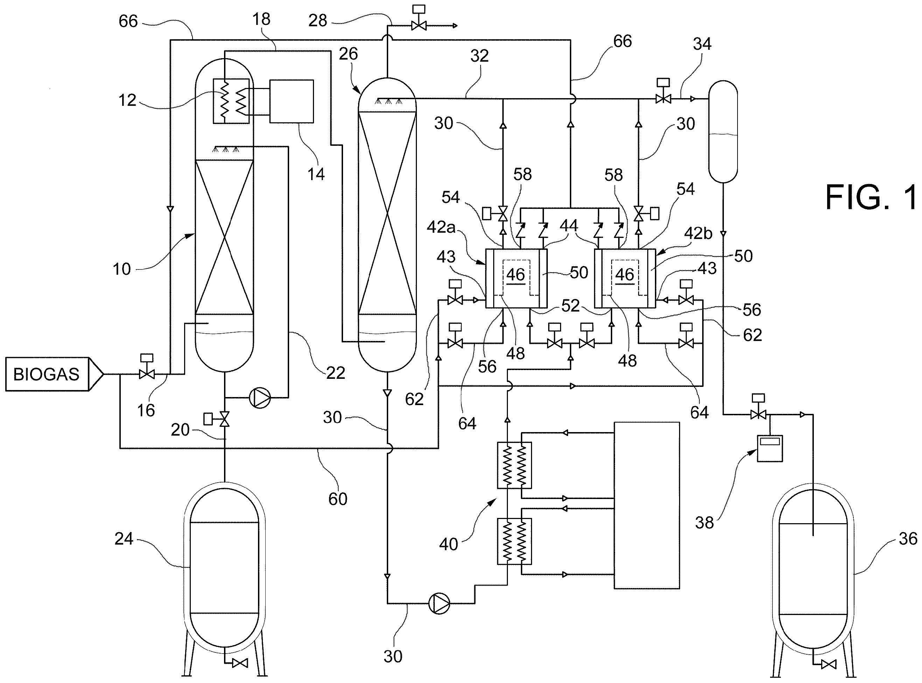

Further advantages and features of the present invention will become apparent from the detailed description that follows, provided by way of non-limiting example with reference to the accompanying drawing, wherein figure 1 is a schematic representation of a plant of the invention.

A plant for upgrading biogas comprises a column 10, typically a tray or packed column, which performs the functions of cooling the biogas and condensing of part of the carbon dioxide contained therein, due to the fact that it incorporates at its top a fluid evaporation coil 12 of a conventional cooling device 14. The column 10 is provided with a biogas feed conduit 16, a head outlet conduit 18, and a bottom outlet conduit 20 from which a recirculating conduit 22 departs, flowing into the upper portion of said column 10. The bottom outlet conduit 20 allows the liquid carbon dioxide condensed in the column 10 to be discharged and flows into a storage tank 24.

The head outlet conduit 18, on the other hand, flows into a liquefaction column 26, also typically a tray or packed column, from the top of which departs a discharge conduit 28 for incondensable gases, and from the bottom of which departs a discharge conduit 30 for a liquid flow, having a recirculation branch 32 which flows into the top portion of the same liquefaction column 26 and an outlet branch 34 flowing into a collection tank 36 for liquid methane, commonly called "biomethane". Moreover, on the branch 34, an analysis device 38 for checking the quality of the biomethane obtained is also provided.

Upstream of the branching of the recirculation branches 32 and the outlet branches 34, a heat exchanger 40 for cooling the liquid flow in the conduit 30 and a filtration device for this liquid flow are arranged in series on the discharge conduit 30 coming from the bottom of the column 26.

The heat exchanger 40 may comprise a number of stages in series, for example from 2 to 4 and in particular 2 as illustrated in the figure, in which a cooling fluid is used at gradually decreasing temperatures. The cooling fluid, typically nitrogen, may be produced by any known frigorific cycle.

The filtration device comprises two units 42a, 42b arranged in parallel, which - as will be described in more detail with reference to the operation of the plant - are intended to be alternately in the operating stage and in the regeneration stage, due to switching the shut-off valves that are opened/closed as desired.

Each filtration unit 42a, 42b comprises a wall or casing delimiting a chamber 46 within which a filtering septum 48 is arranged, and which is surrounded by a jacket 50. The chamber 46 has openings for the entry 52 of the liquid to be filtered and the exit 54 of the filtered liquid during the operating stage, and respectively for the entry 56 and exit 58 of the biogas during the regeneration stage. A further conduit 60, which departs from the biogas feed conduit 16 to the column 10, in turn branches off, for each unit 42a, 42b, into a feed branch 62 of the jacket 50 and a branch 64 connected to the opening for the entry 56 of the chamber 46. In turn, the openings 58 for the exit of the biogas from the chambers 46 (as well as openings 44 for the exit of the biogas from the jackets 50) are connected to a recirculation conduit 66 to the feed conduit 16 of the biogas to the column 10.

In embodiments not illustrated, each filtering unit 42a, 42b could, in turn, be formed of multiple stages in series, possibly operating at different temperatures.

The discharge conduit 30, after passing through the heat exchanger 40, flows into the openings for the entry 52 to the chambers 46, while the openings for the exit 54 are connected to a respective conduit which forms the end part of the discharge conduit 30 before the branching thereof into the recirculation branch 32 directed towards the top of the column 26 and the branch 34 directed towards the liquid biomethane storage tank 36.

The operation of the plant described above is as follows.

The biogas composed mainly of methane and carbon dioxide, which has been produced with any known technology and subjected to conventional desulfurization and dehumidification treatments, is fed into the column 10 at a temperature between 10 and 35°C and a pressure between 30 and 50 bar gauge through the conduit 16.

In the column 10, the biogas is cooled by a countercurrent flow of liquid carbon dioxide, recirculated from the bottom of the column 10, and in the presence of the cooling device 14. The gas flowing out through the conduit 18 may thus reach temperatures between -40 and -55°C. Such cooling also causes the condensation of a consistent fraction of the carbon dioxide originally present in the biogas. The column 10 thus allows a fraction of the carbon dioxide that constitutes a first product of the process of the invention to be separated from the biogas, while the remaining fraction constitutes a component of the gas flow in the head outlet conduit 18, which is correspondingly enriched in methane. As a guideline, about 60-75% of the carbon dioxide originally contained in the biogas is separated by the column 10.

The head outlet conduit 18 flows into the liquefaction column 26 which is held at a temperature between -80 to -150°C and a pressure between 30 to 50 bar gauge. In the column 26, the gas flow coming from the column 10 is placed in countercurrent with a flow of liquid biomethane recirculated from the bottom of the same column 26 through the conduits 30 and 32. From the top of the column 26, a gas flow, consisting mainly of incondensable gases, in particular nitrogen and oxygen accompanied by residues of methane, is discharged through the conduit 28. In the bottom discharge conduit 30 there is a liquid flow consisting mainly of methane and carbon dioxide.

The conduit 30 first passes through the heat exchanger 40, so as to cause the carbon dioxide to solidify as solid particles suspended in the liquid flowing therein, which is cooled to a temperature of -120°C to -150°C.

After this cooling stage, the liquid is fed through the opening 52 into the chamber 46 of the filtration unit of the two filtration units 42a, 42b which is in the operating stage. The septum 48 of the chamber 46 retains the solid carbon dioxide particles, while the liquid - at this point consisting essentially of only methane - flows through the opening 54 into the final part of the conduit 30.

From here, by means of the recirculation branch 32, a fraction of the liquid is recirculated in the head of the liquefaction column 26, while the remaining fraction flows through the outgoing branch 34 towards the storage tank 36, and constitutes the primary product of the process.

As already mentioned, while one filtering unit 42a, 42b is in the operating stage, the other 42b, 42a is in the regeneration stage.

At the beginning of the regeneration stage, the biogas at a temperature between 10 and 35°C coming from the branch 62 of the conduit 60 is made to flow through an opening 43 of the jacket 50 surrounding the chamber 46 of the unit 42b, 42a to be regenerated, so as to start to heat it and cause the evaporation of the liquid biomethane contained therein. The biogas which, passing through the jacket 50, has pre-cooled, is thus sent again through an opening 44 of the jacket 50 and the conduit 66 to the feed conduit 16 of the column 10. Once this preliminary stage has been completed, the biogas coming from the branch 64 of the conduit 60 is made to flow directly through the opening 56 in the chamber 46. This biogas heats the carbon dioxide particles that were retained on the filtering septum 48 during the previous operation stage of the unit 42b, 42a, so as to cause the sublimation thereof. The biogas, cooled by sublimation and enriched with sublimated carbon dioxide, is thus made to exit from the opening 58 of the chamber 46 and sent again via the conduit 66 to the feed conduit 16 of the column 10, thus being integrally recovered.

At the end of regeneration, the unit 42b, 42a may go into the operating stage, while the other unit 42a, 42b may be regenerated, switching their respective roles. As already mentioned, the passage of each filtration unit 42a, 42b from the operating stage to the regeneration stage, as well as the passage between the preliminary stage and the actual regeneration stage, occurs by operating in the desired way the shut-off valves that are fitted to the various conduits, the operation of these valves being well known to a person skilled in the art.

Naturally, without altering the principle of the invention, the details of implementation and embodiments may vary widely with respect to those described purely by way of example, without thereby departing from the scope of the invention as defined in the appended claims.

## Claims

1. Plant for biogas upgrading, including:
- a cooling and condensation column (10) provided with a biogas feed conduit (16), a head outlet conduit (18), and a bottom outlet conduit (20) from which a recirculating conduit (22) departs, flowing into the upper portion of said column (10),
- a liquefaction column (26) into which said head outlet conduit (18) flows, from the top of which departs a discharge conduit (28) for incondensable gases, and from the bottom of which departs a discharge conduit (30) for a liquid flow, which branches into a recirculation branch (32) flowing into the top portion of the liquefaction column (26) and into an outlet branch (34),
- a heat exchanger (40) for cooling the liquid flow in said discharge conduit (30), and
- a filtration device for the liquid flow in said discharge conduit (30), which filtration device retains particles of solid carbon dioxide, is located downstream of the heat exchanger (40) and upstream of the division into a recirculation branch (32) and an outlet branch (34), and comprises at least two units (42a, 42b) arranged in parallel and intended to be used alternately in the operating stage and in the regeneration stage,
wherein each filtration unit (42a, 42b) comprises a wall delimiting a chamber (46) within which a filtering septum (48) is arranged, said chamber (46) being provided with openings for the entry (52) of the liquid to be filtered and the exit (54) of the filtered liquid during the operating stage, and, respectively, for the entry (56) and the exit (58) of the biogas during the regeneration stage.

2. Plant according to claim 1, wherein said chamber (46) is surrounded by a jacket (50) communicating with the biogas feed conduit (16).

3. Plant according to any one of the preceding claims, wherein said heat exchanger (40) comprises a plurality of serially arranged stages, in particular 2 to 4, wherein a cooling fluid at gradually decreasing temperatures is used.

4. Plant according to any one of the preceding claims, wherein said bottom outlet conduit (20) of the cooling and condensation column (10) flows into a storage tank (24) of liquid carbon dioxide, and said outlet branch (34) flows into a storage tank (36) of liquid methane.

5. Plant according to any one of the preceding claims, wherein said cooling and condensation column (10) is cooled by a frigorific apparatus (14) preferably arranged at the top of the column.

6. Process for biogas upgrading by the use of a plant according to any one of the preceding claims, comprising the steps of:
- cooling the biogas with carbon dioxide condensation in said cooling and condensation column (10) maintained at a temperature preferably comprised between -40 and -55°C and a pressure preferably comprised between 30 and 50 bar gauge by mass and heat exchange with a countercurrent flow of liquid carbon dioxide coming from the recirculating conduit (22), so that a liquid carbon dioxide flow is produced in the bottom outlet conduit (20) and a gas flow in the head outlet conduit (18),
- liquefying the gas flow coming from the head outlet conduit (18) in said liquefaction column (26) maintained at a temperature preferably comprised between -120 and -150 °C and a pressure preferably comprised between 30 and 50 bar gauge by heat and mass exchange with a countercurrent flow of liquid methane coming from said recirculation branch (32), so as to produce a flow of incondensable gases in the top discharge conduit (28) and a liquid flow in the bottom discharge conduit (30),
- cooling by the heat exchanger (40) the liquid flow in the discharge conduit (30), so as to cause the formation of solid particles of carbon dioxide suspended in said liquid flow,
- separating by the filtering device said solid particles from said liquid flow, and
- recirculating in the liquefaction column (26) through the recirculation branch (32) a fraction of said liquid flow, while the remaining fraction flows away through the outlet branch (34) into the storage tank (36) of liquid methane, wherein the filtering device comprises two units (42a, 42b) arranged in parallel intended to be alternately used in the operating stage and in the regeneration stage, each unit (42a, 42b) having a chamber (46) with a filtering septum (48) and being provided with openings for the entry (52) of the liquid to be filtered and the exit (54) of the filtered liquid, which are kept open during the operating stage and closed during the regeneration stage, and with openings for the entry (56) and the exit (58) of the biogas, which are kept open during the regeneration stage and closed during the operating stage.

7. Process according to claim 6, wherein, during the regeneration stage of the unit (42a, 42b), biogas is first of all made to flow into the jacket (50) surrounding the chamber (46), and then into the chamber (46), so that the carbon dioxide particles retained on the filtering septum (48) are heated and sublimated.

8. Process according to claim 7, wherein the biogas containing the sublimated carbon dioxide is sent into the biogas feed conduit (16) of the cooling and condensation column (10) and thus integrally recovered.

## Patentansprüche

1. Anlage zur Biogasveredelung, enthaltend:
- eine Kühl- und Kondensationskolonne (10), die mit einer Biogaszufuhrleitung (16), einer Kopfauslassleitung (18) und einer Bodenauslassleitung (20), von der eine rezirkulierende Leitung (22) ausgeht, die in den oberen Teil der Kolonne (10) mündet, ausgestattet ist,
- eine Verflüssigungskolonne (26), in welche die Kopfauslassleitung (18) mündet, von deren Oberseite eine Abführleitung (28) für nicht kondensierbare Gase ausgeht und von deren Unterseite eine Abführleitung (30) für einen Flüssigkeitsstrom ausgeht, die sich in einen rezirkulierenden Zweig (32), der in den oberen Teil der Verflüssigungskolonne (26) mündet, und in einen Auslasszweig (34) verzweigt,
- einen Wärmetauscher (40) zum Kühlen des Flüssigkeitsstroms in der Abführleitung (30), und
- eine Filtervorrichtung für den Flüssigkeitsstrom in der Abführleitung (30), wobei die Filtervorrichtung Partikel aus festen Kohlenstoffdioxid zurückhält, sich stromabwärts des Wärmetauschers (40) und stromaufwärts der Aufteilung in einen rezirkulierenden Zweig (32) und einem Auslasszweig (34) befindet, und mindestens zwei Einheiten (42a, 42b) umfasst, die parallel angeordnet und vorgesehen sind, um abwechselnd in der Betriebsphase und in der Regenerationsphase eingesetzt zu werden,
wobei jede Filtereinheit (42a, 42b) eine Wand umfasst, die eine Kammer (46) abgrenzt, in welcher ein Filterseptum (48) angeordnet ist, wobei die Kammer (46) mit Öffnungen für den Eintritt (52) der zu filternden Flüssigkeit und den Austritt (54) der gefilterten Flüssigkeit während der Betriebsphase, und entsprechend für den Eintritt (56) und den Austritt (58) des Biogases während der Regenerationsphase, ausgestattet ist.

2. Anlage nach Anspruch 1, wobei die Kammer (46) von einem Mantel (50) umgeben ist, der mit der Biogaszufuhrleitung (16) in Verbindung steht.

3. Anlage nach einem der vorhergehenden Ansprüche, wobei der Wärmetauscher (40) eine Vielzahl, insbesondere 2 bis 4, von in Reihe angeordneten Stufen umfasst, wobei ein Kühlfluid mit stufenweise abnehmender Temperatur verwendet wird.

4. Anlage nach einem der vorhergehenden Ansprüche, wobei die Bodenauslassleitung (20) der Kühl- und Kondensationskolonne (10) in einen Speichertank (24) für flüssiges Kohlenstoffdioxid und der Auslasszweig (34) in einen Speichertank (36) für Methan mündet.

5. Anlage nach einem der vorhergehenden Ansprüche, wobei die Kühl- und Kondensationskolonne (10) durch eine bevorzugt am Kopf der Kolonne angeordnete Kühlvorrichtung (14) gekühlt wird.

6. Verfahren zur Biogasveredelung durch Verwendung der Anlage nach einem der vorhergehenden Ansprüche, umfassend die Schritte:
- Kühlen des Biogases durch Kohlenstoffdioxidkondensation in der Kühl- und Kondensationskolonne (10), die bei einer Temperatur von vorzugsweise zwischen -40 und -55°C und einem Druck von vorzugsweise zwischen 30 und 50 bar Überdruck gehalten wird, durch Massen- und Wärmeaustausch mit einem Gegenstrom aus flüssigem Kohlenstoffdioxid, der aus der rezirkulierenden Leitung (22) kommt, um einen flüssigen Kohlenstoffdioxidstrom in der Bodenauslassleitung (20) und ein Gasstrom in der Kopfauslassleitung (18) zu erzeugen,
- Verflüssigen des aus der Kopfauslassleitung (18) kommenden Gasstroms in der Verflüssigungskolonne (26), die bei einer Temperatur von vorzugsweise zwischen -120 und -150°C und einem Druck von vorzugsweise zwischen 30 und 50 bar Überdruck gehalten wird, durch Wärme- und Massenaustausch mit einem Gegenstrom aus flüssigem Methan, der von dem rezirkulierenden Zweig (32) kommt, um einen Strom aus nicht kondensierbaren Gasen in der oberen Abführleitung (28) und einen Flüssigkeitsstrom in der unteren Abführleitung (30) zu erzeugen,
- Kühlen des Flüssigkeitsstroms in der Abführleitung (30) durch den Wärmetauscher (40), um die Bildung von in dem Flüssigkeitsstrom suspendierten Feststoffpartikeln aus Kohlenstoffdioxid zu bewirken,
- Abtrennen der Feststoffpartikel aus dem Flüssigkeitsstrom durch die Filtervorrichtung, und
- Rezirkulieren eines Anteils des Flüssigkeitsstroms in der Verflüssigungskolonne (26) durch den rezirkulierenden Zweig (32), während der restliche Anteil durch den Auslasszweig (34) in den Lagertank (36) für flüssiges Methan abfließt, wobei die Filtervorrichtung zwei Einheiten (42a, 42b) umfasst, die parallel angeordnet und vorgesehen sind, um abwechselnd in der Betriebsphase und der Regenerationsphase eingesetzt zu werden, wobei jede Einheit (42a, 42b) eine Kammer (46) mit einem Filterseptum (48) aufweist, und mit Öffnungen für den Eintritt (52) der zu filternden Flüssigkeit und den Austritt (54) der gefilterten Flüssigkeit, die während der Betriebsphase offen und während der Regenerationsphase geschlossen gehalten werden, und mit Öffnungen für den Eintritt (56) und den Austritt (58) des Biogases, die während der Regenerationsphase offen und während der Betriebsphase geschlossen gehalten werden, ausgestattet ist.

7. Verfahren nach Anspruch 6, wobei während der Regenerationsphase der Einheit (42a, 42b) Biogas zuerst in den Mantel (50), der die Kammer (46) umgibt, und dann in die Kammer (46) geleitet wird, so dass die im Filterseptum (48) zurückgehaltenen Kohlenstoffdioxidpartikel erwärmt und sublimiert werden.

8. Verfahren nach Anspruch 7, wobei das Biogas, welches das sublimierte Kohlenstoffdioxid enthält, in die Biogaszufuhrleitung (16) der Kühl- und Kondensationskolonne (10) geleitet und so wesentlich zurückgewonnen wird.

## Revendications

1. Installation de valorisation de biogaz, comprenant :
- une colonne de refroidissement et de condensation (10) pourvue d'un conduit d'alimentation en biogaz (16), d'un conduit de sortie de tête (18) et d'un conduit de sortie inférieur (20) à partir duquel part un conduit de recirculation (22), en s'écoulant dans la partie supérieure de ladite colonne (10),
- une colonne de liquéfaction (26) dans laquelle ledit conduit de sortie de tête (18) s'écoule, à partir du haut de laquelle part un conduit d'évacuation (28) pour les gaz incondensables, et à partir du bas de laquelle part un conduit d'évacuation (30) pour un écoulement de liquide, qui se ramifie en une ramification de recirculation (32) s'écoulant dans la partie supérieure de la colonne de liquéfaction (26) et en une ramification de sortie (34),
- un échangeur de chaleur (40) pour refroidir l'écoulement de liquide dans ledit conduit d'évacuation (30), et
- un dispositif de filtration pour l'écoulement de liquide dans ledit conduit d'évacuation (30), lequel dispositif de filtration retient les particules de dioxyde de carbone solide, est situé en aval de l'échangeur de chaleur (40) et en amont de la division en une ramification de recirculation (32) et une ramification de sortie (34), et comprend au moins deux unités (42a, 42b) agencées en parallèle et destinées à être utilisées en alternance dans la phase de fonctionnement et dans la phase de régénération,
dans laquelle chaque unité de filtration (42a, 42b) comprend une paroi délimitant une chambre (46) à l'intérieur de laquelle un septum de filtration (48) est agencé, ladite chambre (46) étant pourvue d'ouvertures pour l'entrée (52) du liquide à filtrer et la sortie (54) du liquide filtré pendant la phase de fonctionnement, et, respectivement, pour l'entrée (56) et la sortie (58) du biogaz pendant la phase de régénération.

2. Installation selon la revendication 1, dans laquelle ladite chambre (46) est entourée d'une chemise (50) communiquant avec le conduit d'alimentation en biogaz (16).

3. Installation selon l'une quelconque des revendications précédentes, dans laquelle ledit échangeur de chaleur (40) comprend une pluralité d'étages agencés en série, en particulier 2 à 4, dans lesquels un fluide de refroidissement à des températures diminuant progressivement est utilisé.

4. Installation selon l'une quelconque des revendications précédentes, dans laquelle ledit conduit de sortie inférieur (20) de la colonne de refroidissement et de condensation (10) s'écoule dans une cuve de stockage (24) de dioxyde de carbone liquide, et ladite ramification de sortie (34) s'écoule dans une cuve de stockage (36) de méthane liquide.

5. Installation selon l'une quelconque des revendications précédentes, dans laquelle ladite colonne de refroidissement et de condensation (10) est refroidie par un appareil frigorifique (14) agencé de préférence en haut de la colonne.

6. Procédé de valorisation de biogaz par l'utilisation d'une installation selon l'une quelconque des revendications précédentes, comprenant les étapes consistant à :
- refroidir le biogaz avec condensation de dioxyde de carbone dans ladite colonne de refroidissement et de condensation (10) maintenue à une température comprise de préférence entre -40 et -55 °C et à une pression comprise de préférence entre 30 et 50 bar manométriques par échange de matière et de chaleur avec un écoulement à contre-courant de dioxyde de carbone liquide provenant du conduit de recirculation (22), de sorte qu'un écoulement de dioxyde de carbone liquide est produit dans le conduit de sortie inférieur (20) et un écoulement de gaz dans le conduit de sortie de tête (18),
- liquéfier l'écoulement de gaz provenant du conduit de sortie de tête (18) dans ladite colonne de liquéfaction (26) maintenue à une température comprise de préférence entre -120 et -150 °C et à une pression comprise de préférence entre 30 et 50 bar manométriques par échange de chaleur et de matière avec un écoulement à contre-courant de méthane liquide provenant de ladite ramification de recirculation (32), afin de produire un écoulement de gaz incondensables dans le conduit d'évacuation supérieur (28) et un écoulement de liquide dans le conduit d'évacuation inférieur (30),
- refroidir par l'échangeur de chaleur (40) l'écoulement de liquide dans le conduit d'évacuation (30), afin de provoquer la formation de particules solides de dioxyde de carbone en suspension dans ledit écoulement de liquide,
- séparer par le dispositif de filtration lesdites particules solides dudit écoulement de liquide, et
- remettre en recirculation dans la colonne de liquéfaction (26) à travers la ramification de recirculation (32) une fraction dudit écoulement de liquide, tandis que la fraction restante s'écoule à travers la ramification de sortie (34) dans la cuve de stockage (36) de méthane liquide, dans lequel le dispositif de filtration comprend deux unités (42a, 42b) agencées en parallèle destinées à être utilisées en alternance dans la phase de fonctionnement et dans la phase de régénération, chaque unité (42a, 42b) comportant une chambre (46) avec un septum de filtration (48) et étant pourvue d'ouvertures pour l'entrée (52) du liquide à filtrer et la sortie (54) du liquide filtré, lesquelles sont maintenues ouvertes pendant la phase de fonctionnement et fermées pendant la phase de régénération, et d'ouvertures pour l'entrée (56) et la sortie (58) du biogaz, lesquelles sont maintenues ouvertes pendant la phase de régénération et fermées pendant la phase de fonctionnement.

7. Procédé selon la revendication 6, dans lequel, pendant la phase de régénération de l'unité (42a, 42b), du biogaz est tout d'abord amené à s'écouler dans la chemise (50) entourant la chambre (46), puis dans la chambre (46), de sorte que les particules de dioxyde de carbone retenues sur le septum de filtration (48) sont chauffées et sublimées.

8. Procédé selon la revendication 7, dans lequel le biogaz contenant le dioxyde de carbone sublimé est envoyé dans le conduit d'alimentation en biogaz (16) de la colonne de refroidissement et de condensation (10) et ainsi récupéré intégralement.
